# Europäisches Patentamt

# European Patent Office

# Office européen des brevets

(11) Publication number: **0 025 721**
**B1**

(12) **EUROPEAN PATENT SPECIFICATION**

(45) Date of publication of patent specification: **01.02.84**

(51) Int. Cl.³: **A 61 K 33/26** //(A61K33/26, 33/08)

(21) Application number: **80303259.8**

(22) Date of filing: **16.09.80**

(54) **A medication for oral administration and process for its manufacture.**

(30) Priority: **18.09.79 AU 530/79**

(43) Date of publication of application:
**25.03.81 Bulletin 81/12**

(45) Publication of the grant of the patent:
**01.02.84 Bulletin 84/5**

(84) Designated Contracting States:
**AT BE CH DE FR GB IT LI LU NL SE**

(56) References cited:
**DE - C - 20 925**
**GB - A - 768 452**
**GB - A - 1 518 364**
**US - A - 3 660 570**

**MODERN DRUG ENCYCLOPEDIA, 8th ed. 1961,**
**The Reuben H. Donelley Corp. New York, US**
**UNLISTED DRUGS, vol. 14, August 1962,**
**Chatham N.J. USA**
**ROTE LISTE 1961, Editio Cantor**
**Aulendorf/Württ. DE**
**DICTIONNAIRE VIDAL, 1973, OVP, Paris, FR**

(73) Proprietor: **HOLLAND, Charles Cottrell**
**Box 113 Post Office**
**Sawtell New South Wales 2452 (AU)**

(72) Inventor: **HOLLAND, Charles Cottrell**
**Box 113 Post Office**
**Sawtell New South Wales 2452 (AU)**

(74) Representative: **Eyles, Christopher Thomas et al,**
**BATCHELLOR, KIRK & EYLES 2 Pear Tree Court**
**Farringdon Road**
**London, EC1R 0DS (GB)**

Courier Press, Leamington Spa, England.

# 0 025 721

A medication for oral administration and process for its manufacture

This invention relates to a preparation useful for the treatment of rheumatoid arthritis and like disorders and a process for the production of the preparation.

Rheumatoid arthritis is well known as are its clinical features which usually include a prodromal illness lasting for weeks or months in which fatigue, malaise and pains and stiffness in the limbs are common. The prodromal illness is followed by the appearance of arthritis, essentially peripheral polyarthritis. This usually begins in the metacarpa-phalangel or interphalangel joints of the hands and feet and spreads proximally to involve the wrists and elbows, ankles and knees. Shoulders and hips may likewise be affected. Affected joints are tender, swollen and hot and movement is painful.

Although there is no definitive work as to the cause of rheumatoid arthritis, it is probable that it is initiated by circulating immune complexes. The immune response of the body is initiated by foreign antigens which are usually associated with pathogenic organisms or their products. Antigenic compounds may be complex proteins or carbohydrates of large molecular size.

Many antigens which initiate a specific antibody response have been observed to undergo phagocytosis by tissue macrophages. These cells do not synthesise antibodies but appear to participate in the immune response by concentrating antigen and passing it to adjacent lymphocytes. After antigenic stimulation they divide and differentiate into plasma cells which contain and secrete antibody molecules.

Serum antibodies are confined to the gamma-globulin fraction and are referred to as immunoglobulins (Ig). They are large proteins containing two heavy and two light polypeptide chains. The different classes of immunoglobulin (IgG, IgA, IgM, IgD and IgE) are distinguished by the structure of their heavy chains. Each Ig chain is made up of repeating units of about seventy amino acids enclosed by a disulphide bond. Antibodies have evolved to perform two distinct functions which are separately localized in the molecule: each antibody combines with a specific antigen and antibodies carry out various effector functions which are consequent upon interaction with complement or with specific cells or membranes of the body.

Clinical disorders may be associated with either diminished or increased antibody production. An excessive production of Ig leading to a diffuse increase of gamma-globulin is seen in many clinical disorders. Humoral antibodies are of fundamental clinical importance particularly in a wide range of pathological states involving either immediate hypersensitivity reactions, auto-antibody formation or reactions to circulating immune complexes. Immune reactions involve the primary interaction of antibody with the pathogen or its toxic products.

Antigen-antibody complexes present in the circulation may form micro-precipitates in small blood vessels and lead to accumulation of polymorphonuclear leucocytes, vascular occlusion and perivascular inflammation. Such a mechanism is known as a circulating immune complex mechanism. The most obvious disorder of immunity in rheumatoid arthritis is the presence in the serum of most patients of what is called the "rheumatoid factor". This is an IgM globulin which will react with human IgG antigen provided that antigen is aggregated or fixed to particles such as red cells.

Evidence suggests, however, that the rheumatoid factor does not cause rheumatoid arthritis, but develops as a consequence of the disease. It seems probable that rheumatoid arthritis is initiated by circulating immune complexes.

Hitherto, treatment for rheumatoid arthritis has centred upon such specific measures as doses of salicylates such as Indomethacin and Phenylbutazone, steroid hormones and Chloroquinine. Generally, prognoses is parther guarded until the rate of progress of the disease in a particular patient has been assessed and it may be said that those whose symptoms start very early or very late in life do worst and those in the middle do best.

It has been found that the preparations of the invention have been particularly effective in the treatment of rheumatoid arthritis and related disorders and although no definite explanation for this has yet emerged it would seem that the components of the preparations exhibit some synergistic effect which either inhibits the initiation of rheumatoid arthritis by the circulating immune complexes, or inhibits the formation of the immune complexes, or sets up a mechanism which counter-balances or overcomes the mechanism which leads to the disease.

According to the present invention there is provided a preparation for oral administration for the treatment of rheumatoid arthritis which comprises an aqueous solution containing calcium ions, the calcium ions being present in an amount of up to 172 milligrams per litre and ferrous ions, the ferrous ions being present in an amount of 0.01 to 0.4 milligrams per litre. Preferably the solution contains 12 to 24 milligrams per litre of calcium ions and 0.1 to 0.3 milligrams per litre of ferrous ions.

In a preferred form the invention provides a preparation for oral administration for the treatment of rheumatoid arthritis which comprises an aqueous solution of calcium oxide and ferrous sulphate, the calcium oxide being present in an amount of up to 120 milligrams per litre and the ferrous sulphate from 0.5 to 1.5 milligrams per litre.

A particularly useful preparation according to the invention comprises an aqueous solution

2

containing calcium ions in an amount of about 12 milligrams per litre and ferrous ions in an amount of about 0.2 milligrams per litre.

A preparation according to the invention may also include traces of aluminium, lead, arsenic, cadmium, chromium, nickel, manganese and magnesium. These traces may be present in the following amounts:—

| | |
|---|---|
| aluminium | less than 0.5 ppm |
| lead | about 0.1 ppm |
| arsenic | less than 0.04 ppm |
| cadmium | less than 0.1 ppm |
| chromium | about 0.1 ppm |
| nickel | about 0.2 ppm |
| manganese | about 13 ppm |
| magnesium | about 7 ppm |

A process for the production of a preparation according to the invention comprises the steps of:—

(i) mixing commercial lime (as hereinafter defined) with water and then decanting the supernatant liquid which constitutes component A,
(ii) mixing commercial ferrous sulphate (as hereinafter defined) with water and decanting the supernatant liquid which constitutes component B,
(iii) mixing components A and B with or without additional water such that the resultant solution contains 12 to 24 milligrams per litre of calcium ions and 0.1 to 0.3 milligrams per litre of ferrous ions.

The mixture of lime and water may be left to stand for at least eight hours (preferably twelve hours) before the supernatant liquid is decanted.

A method of treating rheumatoid arthritis and related disorders comprises a dosage of five millilitres of a preparation which comprises an aqueous solution containing up to 172 milligrams per litre of calcium ions and 0.01 to 0.4 milligrams per litre of ferrous ions which is administered orally to a patient every seven days.

The invention is illustrated by the following examples:—

Example 1

An aqueous solution containing 86 milligrams per litre of calcium ions and 0.2 milligrams per litre of ferrous ions was prepared as follows:—

Commercial lime was mixed with water in the ratio of 0.9 kilograms of lime to 4.55 litres of water to form a saturated solution which upon chemical analysis was shown to contain:

| | | | |
|---|---|---|---|
| Fe | less than 0.05 ppm ($\mu$g/ml) | Cd | less than 0.05 ppm |
| Ca | 70 ppm | Cr | less than 0.1 ppm |
| Mg | 7 ppm | Ni | 0.2 ppm |
| Al | less than 0.5 ppm | Mn | less than 0.05 ppm |
| Pb | less than 0.1 ppm | $SO_4$ | less than 0.2 ppm |
| As | less than 0.02 ppm | Cl | 7 ppm |

As used throughout the specification and claims the term "commercial lime" means non-pure calcium oxide such as what is commonly called agricultural lime which contains a number of trace elements. Although it is by no means clear, it may be that these trace elements collectively or separately contribute to the synergistic effect exhibited by the preparations of the invention.

A typical commercial lime has been analysed as follows:—

| | |
|---|---|
| Moisture | 0.04% |
| Loss on ignition | 42.0% |
| Acid insoluble ($SiO_2$) | 0.8% |
| $Fe_2O_3$ | 0.09% |
| $Al_2O_3$ | 0.07% |
| CaO | 43.2% |
| MgO | 0.5% |
| Pb | 30 ppm ($\mu$g/g) |
| Cd | less than 5 ppm |
| Cr | 25 ppm |
| Mn | 117 ppm |
| As | less than 2 ppm |
| Ni | 16 ppm |
| $SO_4$ | Not detected |
| Cl | Not detected |

3

After the lime/water mixture had stood for twelve hours, the supernatant liquid was decanted to provide component A.

Commercial ferrous sulphate (ferrous sulphate heptahydrate) was mixed with water in the ratio of 28.3 grams of ferrous sulphate to 4.55 litres of water to form a solution which upon chemical analysis was shown to contain:—

| | |
|---|---|
| Fe | 850 ppm ($\mu$g/ml) |
| $SO_4$ | 1600 ppm |
| As | less than 0.02 ppm |
| Cr | 0.1 ppm |
| Mn | 13 ppm |
| Pb | 0.1 ppm |
| Cd | less than 0.05 ppm |

As used in the specification and claims the term "commercial ferrous sulphate" means non-pure ferrous sulphate heptahydrate such as what is commonly called agricultural ferrous sulphate which contains trace elements such as arsenic, lead, cadmium, chromium and manganese as indicated above. These trace elements may collectively or separately contribute to the working of the invention.

An analysis of a typical commercial ferrous sulphate shows the above trace elements and:—

| | |
|---|---|
| Ferrous sulphate heptahydrate | 94—96% |
| Free water | 6—4% |
| Total iron (Fe) | 18.9—19.3% |
| Total sulphur | |
| as $SO_4$ | 32.5—34% |
| as S | 10.8—11.1% |

and a 5% solution has a pH not less than 2.4.

The supernatant liquid was decanted to provide component B.

Appropriate quantities of components A and B were then mixed to provide an aqueous solution which when analysed by an atomic absorption spectrometer contained:—

| | |
|---|---|
| Calcium ion ($Ca^+$) | 86 mg/l |
| Ferrous ion ($Fe^{++}$) | 0.2 mg/l |

The above analysis corresponds to:—

| | |
|---|---|
| Calcium oxide (CaO) | 120 mg/l |
| Ferrous sulphate hepta-hydrate ($FeSO_4 . 7H_2O$) | 0.996 mg/l |

In therapeutic product terms, the analysis indicates that each five millilitres contains:—

| | |
|---|---|
| Calcium oxide | 1.2 milligrams |
| Ferrous sulphate | 10 micrograms |

Example 2

Using the procedures as set forth in Example 1, another solution was prepared with the same component B but the component A was diluted so that the solution contained 12 mg/l of calcium ions, i.e., eave five millilitres contain 85 mcg of calcium oxide.

Various experiments have been conducted on patients suffering from rheumatoid arthritis using dosages of the preparations of the present invention and the following results were obtained:—

4

# 0 025 721

## TABLE 1

| Patient | Centre of rheumatoid arthritis | Period of suffering | Period of treatment |
|---|---|---|---|
| A | back, hips, neck | 10 years | 20 weeks |
| B | knees, hips | many years | 2 weeks |
| C | hands, neck, leg joints and other parts | 35 years | 4 months, break of 4 weeks then 3 months |
| D | over back, shoulders, legs, arms | over 5 years | 4 months |
| E | neck joints | not sure— many years? | 2 months |
| F | back, arms, hards, shoulders | 10 years | 4 months |
| G | hands, arms, shoulders | 6—7 years | 4 months |
| H | hips | 32 years | 3 months |
| I | legs and shoulders | many years | 2 months |

Note: In each case the treatment was one dose of 5 millilitres each week.

All the patients experienced relief after a weekly dose of the preparation extending over four weeks. Mild cases (patients B, E and I) were effectively cured in four to eight weeks. The more severe cases (patients A and C) required longer treatment of up to twenty weeks whereafter occasional doses kept the disease in check.

## Claims

1. A preparation for oral administration for the treatment of rheumatoid arthritis which comprises an aqueous solution containing calcium ions, the calcium ions being present in an amount of up to 172 milligrams per litre and ferrous ions, the ferrous ions being present in an amount of 0.01 to 0.4 milligrams per litre.

2. A preparation according to Claim 1, wherein said aqueous solution contains 12 to 24 milligrams per litre of calcium ions and 0.1 to 0.3 milligrams per litre of ferrous ions.

3. A preparation according to Claim 2, wherein said aqueous solution contains calcium ions in an amount of about 12 milligrams per litre and ferrous ions in an amount of about 0.2 milligrams per litre.

4. A preparation according to claim 1 which comprises an aqueous solution of calcium oxide and ferrous sulphate, the calcium oxide being present in an amount of up to 120 milligrams per litre and the ferrous sulphate from 0.5 to 1.5 milligrams per litre.

5. A preparation according to Claim 4, wherein said calcium oxide is present in an amount of about 17 milligrams per litre and said ferrous sulphate is present in an amount of about 0.996 milligrams per litre.

6. A preparation according to any one of the preceding claims and further including traces of aluminium, lead, arsenic, cadmium, chromium, nickel, manganese and magnesium.

7. A preparation according to any one of Claims 1 to 5 and further including:—

| | |
|---|---|
| aluminium | less than 0.5 ppm |
| lead | about 0.1 ppm |
| arsenic | less than 0.04 ppm |
| cadmium | less than 0.1 ppm |
| chromium | about 0.1 ppm |
| nickel | about 0.2 ppm |
| manganese | about 13 ppm |
| magnesium | about 7 ppm |

8. A process for the production of the preparation according to Claim 2 comprising the steps of:—

(i) mixing commercial lime with water, said commercial lime comprising non pure calcium oxide, and then decanting the supernatant liquid which constitutes component A,

(ii) mixing commercial ferrous sulphate with water, said commercial ferrous sulphate comprising non pure ferrous sulphate heptahydrate, and decanting the supernatant liquid which constitutes component B,

5

(iii) mixing components A and B with or without additional water such that the resultant solution contains 12 to 24 milligrams per litre of calcium ions and 0.1 to 0.3 milligrams per litre of ferrous ions.

9. A process according to Claim 8 wherein the mixture of lime and water is a saturated solution.

10. A process according to Claim 8 or Claim 9 wherein the mixture of lime and water is left to stand for at least eight hours before the supernatant liquid is decanted.

11. A process according to Claim 8 or Claim 9 wherein the mixture of lime and water is left to stand for twelve hours before the supernatant liquid is decanted.

## Revendications

1. Préparation pour l'administration orale pour le traitement de l'arthrite rheumatoïde caractérisée en ce qu'elle comprend une solution aqueuse contenant des ions de calcium, les ions de calcium étant présents dans une proportion de l'ordre de 172 milligrammes par litre et des ions de fer, les ions de fer étant présents dans une proportion 0.04 milligrammes par litre.

2. Préparation selon la revendication 1, caractérisée en ce que la solution aqueuse contient 12 à 14 milligrammes par litre d'ions de calcium et 0.1 à 0.3 milligrammes par litre d'ions ferreux.

3. Préparation selon la revendication 2, caractérisée en ce que la solution aqueuse contient des ions de calcium dans une proportion de l'ordre de 12 milligrammes par litre et des ions de fer dans une proportion de l'ordre de 0.2 milligrammes par litre.

4. Préparation selon la revendication 1, caractérisée en ce qu'elle comprend une solution aqueuse d'oxyde de calcium et de sulphate ferreux, l'oxyde de calcium étant présent dans une proportion de l'ordre de 120 milligrammes par litre et le sulphate ferreux dans une proportion de 0.5 à 1.5 milligrammes par litre.

5. Préparation selon la revendication 4, caractérisée en ce que l'oxyde de calcium est présent dans une proportion de l'ordre de 17 milligrammes par litre et le sulphate ferreux est présent dans une proportion de l'ordre de 0.996 milligrammes par litre.

6. Préparation suivant l'une quelconque des revendications précédentes, caractérisée en ce qu'elle comprend de plus, des traces d'aluminium, de plomb, d'arsenic, de cadmium, de chrome, de nickel, de manganèse et de magnésium.

7. Préparation suivant l'une quelconque des revendications 1 à 5, caractérisée en ce qu'elle comprend:

| | |
|---|---|
| Aluminium | moins de 0.5 ppm |
| Plomb | de l'ordre de 0.1 ppm |
| Arsénic | moins de 0.04 ppm |
| Cadmium | moins de 0.1 ppm |
| Chrome | de l'ordre de 0.1 ppm |
| Nickel | de l'ordre de 0.2 ppm |
| Manganèse | de l'ordre de 13 ppm |
| Magnésium | de l'ordre de 7 ppm |

8. Procédé pour la production de la préparation selon la revendication 2, caractérisé en ce qu'il comprend les phases suivantes:

a) on mélange de la chaux commerciale avec de l'eau, cette chaux commerciale comprenant de l'oxyde de caclium impur et en fait décanter le liquide surnageant qui constitue le composant A;

b) on mélange du sulphate ferreux commercial avec de l'eau, ce sulphate ferreux commercial comprenant du sulphate heptahydraté ferreux non pur et on décante le liquide surnageant lequel constitue le composant B.

c) on mélange les composants A et B avec ou sans eau additionnelle de manière que la solution résultante contienne 12 à 14 milligrammes par litre d'ions de calcium et 0.1 à 0.3 milligrammes par litre d'ions ferreux.

9. Procédé suivant la revendication 8, caractérisé en ce que le mélange de chaux et d'eau est une solution saturée.

10. Procédé selon les revendications 8 et 9, caractérisé en ce que le mélange de chaux et d'eau est laissé reposé au moins huit heures avant que le liquide surnageant soit décanté.

11. Procédé selon les revendications 8 et 9, caractérisé en ce que le mélange de chaux et d'eau est laissé reposé douzes heures avant que le liquide surnageant soit décanté.

## Patentansprüche

1. Präparat zur oralen Verabreichung für die Behandlung von rheumatoider Arthritis, das eine Calcium- und Eisen(II)-Ionen enthaltende, wässrige Lösung aufweist, wobei die Calcium-Ionen in einer

**0 025 721**

Menge bis zu 172 Milligramm je Liter und die Eisen(II)-Ionen in einer Menge von 0,01 bis 0,4 Milligramm je Liter vorliegen.

2. Präparat nach Anspruch 1, bei dem die wässrige Lösung 12 bis 24 Milligramm Calcium-Ionen je Liter und 0,1 bis 0,3 Milligramm Eisen(II)-Ionen je Liter enthält.

3. Präparat nach Anspruch 2, bei dem die wässrige Lösung Calcium-Ionen in einer Menge von etwa 12 Milligramm je Liter und Eisen(II)-Ionen in einer Menge von etwa 0,2 Milligramm je Liter enthält.

4. Präparat nach Anspruch 1, das eine wässrige Lösung von Calciumoxid und Eisen(II)-Sulfat umfaßt, wobei das Calciumoxid in einer Menge von bis zu 120 Milligramm je Liter und das Eisen(II)-Sulfat von 0,5 bis 1,5 Milligramm je Liter vorliegt.

5. Präparat nach Anspruch 4, bei den das Calciumoxid in einer Menge von etwa 17 Milligramm je Liter und das Eisen(II)-Sulfat in einer Menge von etwa 0,996 Milligramm je Liter vorliegt.

6. Präparat nach einem der vorherigen Ansprüche, das ferner Spuren von Aluminium, Blei, Arsen, Kadmium, Chrom, Nickel, Mangan und Magnesium umfaßt.

7. Präparat nach einem der Ansprüche 1 bis 5, das ferner weniger als 0,5 ppm Aluminium, etwa 0,1 ppm Blei, weniger als 0,04 ppm Arsen, weniger als 0,1 ppm Kadmium, etwa 0,1 ppm Chrom, etwa 0,2 ppm Nickel, etwa 13 ppm Mangan und etwa 7 ppm Magnesium aufweist.

8. Verfahren zur Herstellung des Präparats nach Anspruch 2, das folgende Stufen umfaßt:

a) Mischung von handelsüblichem Kalk, der unreines Calciumoxid aufweist, mit Wasser und dann Dekantierung der überstehenden Flüssigkeit, die Bestandteil A bildet;

b) Mischung von handelsüblichem Eisen(II)-Sulfat, das unreines Eisen(II)-Sulfat-Heptahydrat auf weist, mit Wasser und Dekantierung der überstehenden Flüssigkeit, die Bestandteil B bildet;

c) Mischung der Bestandteile A und B mit oder ohne zusätzlichem Wasser, so daß die resultierende Lösung 12 bis 24 Milligramm Calcium-Ionen je Liter und 0,1 bis 0,3 Milligramm Eisen(II)-Ionen je Liter enthält.

9. Verfahren nach Anspruch 8, bei dem das Gemisch aus Kalk und Wasser eine gesättigte Lösung ist.

10. Verfahren nach Anspruch 8 oder 9, bei dem das Gemisch aus Kalk und Wasser wenigstens acht Stunden stehen gelassen wird, bevor man die überstehende Flüssigkeit dekantiert.

11. Verfahren nach Anspruch 8 oder 9, bei dem das Gemisch aus Kalk und Wasser zwölf Stunden stehen gelassen wird, bevor man die überstehende Flüssigkeit dekantiert.